# EUROPEAN PATENT SPECIFICATION

(11) **EP 2 954 325 B1**
(45) Date of publication and mention of the grant of the patent: **11.09.2019**
(21) Application number: 14706070.1
(22) Date of filing: 05.02.2014
(51) Int. Cl.: G01N 33/564

(54) **DIAGNOSIS OF RHEUMATOID ARTHRITIS**
DIAGNOSE VON RHEUMATOIDER ARTHRITIS
DIAGNOSTIC DE POLYARTHRITE RHUMATOÏDE

(30) Priority: 05.02.2013 GB 201302036
(43) Date of publication of application: 16.12.2015
(73) Proprietor: Queen Mary University of London, London Greater London E1 4NS (GB); The University Of Exeter, Exeter, Devon EX4 4QJ (GB); King's College London, London WC2R 2LS (GB)
(72) Inventor: NISSIM, Ahuva, London EC1M 6BQ (GB); PERRETT, David, London EC1M 6BQ (GB); WINYARD, Paul G., Exeter EX1 2LU (GB); CORRIGALL, Valerie M., London SE1 1UL (GB); PANAYI, Gabriel S., London SE1 1UL (GB)
(74) Representative: Lau, Sarah Jane
(86) International application number: PCT/GB2014/050333
(87) International publication number: WO 2014/122456

(56) References cited:
- WO-A1-2007/017556
- WO-A1-2012/156313
- WO-A2-2010/115745
- AHUVA NISSIM ET AL: "Generation of Neoantigenic Epitopes After Posttranslational Modification of Type II Collagen by Factors Present Within the Inflamed Joint", ARTHRITIS & RHEUMATISM, J.B. LIPPINCOTT CO, vol. 52, no. 12, 1 December 2005 (2005-12-01), pages 3829-3838, XP007905787, ISSN: 0004-3591, DOI: 10.1002/ART.21479
- PRUIJN GER JM ET AL: "The use of citrullinated peptides and proteins for the diagnosis of rheumatoid arthritis", ARTHRITIS RESEARCH & THERAPY 2010 LNKD- PUBMED:20236483,, vol. 12, no. 1, 1 January 2010 (2010-01-01), page 203, XP055107982,
- STROLLO ROCKY ET AL: "Autoantibodies to Posttranslationally Modified Type II Collagen as Potential Biomarkers for Rheumatoid Arthritis", ARTHRITIS & RHEUMATISM, vol. 65, no. 7, July 2013 (2013-07), pages 1702-1712, XP055108004,

## Description

### Field of the Invention

The present invention relates to biomarkers for rheumatoid arthritis (RA). The invention specifically relates to the use of such biomarkers in methods for diagnosing RA and predicting the response of patients with RA to drugs.

### Background to the Invention

Rheumatoid arthritis (RA) is the most common autoimmune chronic arthritis and affects 0.5 to 1% of the population. This disease is characterized by chronic inflammation of the joints and is associated with synovitis and erosion of the cartilage and bone. The damage involves the action of pro-inflammatory cytokines, free radicals and matrix metalloproteinases (MMP). The high influx of metabolically active immune cells infiltrating the inflamed joints consumes increased amounts of oxygen, in association with respiratory burst and the generation of reactive oxidants. Some of the key reactive oxygen species (ROS) present in inflamed joints are superoxide radical (O₂˙⁻), hydrogen peroxide (H₂O₂), lipid hydroperoxides (LOOH), hydroxyl radical (˙OH), hypochlorous acid (HOCl), nitric oxide (NO˙) and peroxynitrite (ONOO⁻), which are involved in acute and chronic inflammation. In addition, cartilage damage as a result of collagen oxidation by glycation and formation of advanced glycation end-products (AGE) are evident despite the absence of hyperglycemia.

Although osteoarthritis (OA) is not a systemic inflammatory disease, synovial inflammation is prevalent albeit not as severe as in RA. Chondrocytes, synoviocytes and infiltrating immune cells produce similar inflammatory mediators in an OA joint to those present in inflamed RA joints. OA chondrocytes are metabolically active and produce high levels of ROS. In fact, in both OA and RA, cartilage damage as a result of collagen oxidation by glycation and formation of advanced glycation end-products (AGE) is evident.

Collagen type II (CII) is a principal component of human articular cartilage. Thus this protein is a prominent target for chemical post-translational modification by ROS in inflamed joints, giving rise to the product CII post-translationally modified by ROS (ROS-CII). Native CII is a well-studied auto-antigen in RA, Nevertheless, the present inventors have previously reported auto-immune reactivity against ROS-CII (Nissim et al. Arthritis & Rheumatism 52: 3829-38, 2005). Distinct from chemical post-translational modifications, the relevance of enzymatic post-translational modifications in modulating the immune response in RA has been demonstrated. Antibodies against cyclic citrullinated peptides (ACPA) and proteins have become important diagnostic and prognostic tools in RA (Pruijn et al. Arthritis Research & Therapy 12(1): 203, 2010). Notably, citrullinated CII is also part of the ACPA reactivity in many patients with RA. The origin of the citrullinated protein and its contribution to disease pathogenesis are, however, still incompletely understood. In addition, the diagnostic sensitivity of ACPA is approximately 60%, with some centres reporting as low as 40% ACPA positivity at the time of diagnosis. In any case a significant percentage of RA patients are ACPA negative. Furthermore, levels of ACPA do not change significantly during disease progression, even after treatment. Therefore, there is a need in the art for new methods for improving diagnosis and prognosis in RA patients.

### Summary of the Invention

The present inventors have established that auto-antibodies to oxidised CII, for example ROS-CII, can be used as biomarkers for RA. In particular, the presence of antibodies against oxidised CII in a patient can be used in the diagnosis of RA. They can also be used to predict the response of patients with RA to certain drugs.

Accordingly, in a first aspect, the present invention provides a method of diagnosing rheumatoid arthritis (RA), comprising:
testing a sample from a subject for the presence or absence of antibodies against oxidised collagen II;
wherein the presence of antibodies against oxidised collagen II in the sample is indicative of RA in the subject, and wherein the subject does not have antibodies to cyclic citrullinated peptides (ACPA).

### Detailed Description of the Invention

In a first aspect, the present invention relates to a method for diagnosing RA. In other words, the invention relates to a method of determining whether a subject has RA or, alternatively, a method of testing for RA. The method of the invention can also be described as an assay. The inventors have found that the method of the first aspect of the invention can be used to accurately diagnose RA at an early stage, for example upon onset of the disease (for example as determined by clinical synovitis onset) but typically within the first year of the disease developing. The method is therefore typically carried out on a sample from a subject who is suspected of having RA. The subject may, for example, be exhibiting one or more symptoms of RA such as joint pain (arthralgia), swollen joints (for example joint effusion), synovitis (inflammation of the synovial membrane), musculoskeletal pain, stiffness in the joints, fatigue, irritability, depression, anaemia and/or flu-like symptoms. The method is typically carried out on a sample from a subject who has not previously been treated with any drugs for RA, such as disease-modifying anti-rheumatic drugs (DMARDs) or biologics.

The inventors have also surprisingly found that the presence of antibodies against oxidised CII in a subject as a diagnostic tool for RA is independent of the presence of antibodies against cyclic citrullinated peptides (ACPA) in the subject or any other classical existing diagnostic test of RA such as C reactive protein (CRP), DAS28 or rheumatoid factor (RF). The method of the first aspect of the invention therefore finds use in the diagnosis of patients who do not have antibodies to cyclic citrullinated peptides (ACPA), i.e. are ACPA negative.

Tests to determine the presence or absence of ACPA in patients are known in the art. These include the second-generation CCP test (CCP2 test). Several such tests are commercially available, for example from Diastat™ from Axis-Shield Diagnostics Limited, Dundee, UK, Immunoscan-CCP Plus™ from Eurodiagnostica, Malmö, Sweden, ELIA-CCP™ from Phadia, and Quanta Lite from Inova. ACPA assays are described, for example, in Pruijn et al., Arthritis Research & Therapy, 12(1):203 (2010).

As described herein, ACPA have become important diagnostic and prognostic tools in RA, but the diagnostic sensitivity of ACPA is not high (∼ 60%). The present invention therefore provides a new method for improved diagnosis of RA, because it can be used to diagnose patients who are ACPA negative.

The method of the first aspect of the invention involves testing a sample from a subject for the presence or absence of antibodies against oxidised collagen II (CII).

Oxidised collagen II (CII) is post-translationally modified collagen II (CII) that has been oxidised by non-enzymatic glycation or by reactive oxygen species (ROS) or by reactions involving aldehydes or a combination thereof. Reactive oxygen species include, but are not limited to, for example, superoxide radical (O₂˙⁻), hydrogen peroxide (H₂O₂), lipid hydroperoxides (LOOH), lipid hydroperoxides in combination with transition metal ions (e.g. ferrous, ferric, cuprous or cupric salts), hydroxyl radical (˙OH), which is often generated in biological systems by hydrogen peroxide in combination with transition metal ions (e.g. ferrous, ferric, cuprous or cupric salts), hypochlorous acid (HOCl), hypobromous acid (HOBr), nitric oxide (NO˙), nitrogen dioxide (NO₂˙), and peroxynitrite (ONOO⁻). Some researchers in the field use terms such as "reactive nitrogen species" (e.g. NO˙) and "reactive chlorine species" (e.g. HOCl) to distinguish subgroups of ROS, but in this document we are using the term ROS in its broadest sense. Glycation is typically induced by glucose, ribose or by other sugars. CII can also be chemically modified by reactions involving aldehydes, for example malonaldehyde and/or 4-hydroxynonenal.

Typically, the oxidised collagen II (CII) is post-translationally modified collagen II (CII) that has been oxidised by reactive oxygen species (ROS), i.e. ROS-CII. In this embodiment of the invention, the ROS is typically hypochlorous acid (HOCl). The term "oxidised collagen II" as used herein also includes fragments of oxidised collagen II, i.e. fragments of CII that have been modified by ROS or by non-enzymatic glycation or any other method described herein. Such fragments modified by ROS are referred to herein as "ROS-fCII". The present invention therefore involves testing a sample from a subject for the presence or absence of antibodies against oxidised collagen II (CII) or a fragment thereof. Fragments of oxidised CII can, for example, be generated by cleavage of CII with matrix metalloproteinases (MMPs) or ROS and can be further be modified by additional ROS.

The method involves testing a sample from a subject for the presence or absence of antibodies against oxidised collagen II (CII). The antibodies to be tested for are auto-antibodies against oxidised CII that are present in the subject. As such the antibodies are typically of the immunoglobulin G (IgG) isotype or alternatively of the IgM or IgA isotype. The sample can be any sample from a subject that could contain auto-antibodies against oxidised collagen II. Typically, the sample is a sample of synovial fluid (SF) or serum, plasma or whole blood. In a preferred embodiment, the sample is a serum or SF sample. In alternative embodiments, the sample is a sample of immune cells infiltrating to the inflamed joints, a tissue sample from a cartilage biopsy or a synovial membrane biopsy.

The presence or absence of antibodies against oxidised CII can be determined by any suitable method or assay. There is a wide range of different types of immunoassays available that can be used to measure autoantibodies in a sample. Typically, the presence or absence of such antibodies is determined using an assay based on antibody-antigen binding such as an ELISA assay or Western Blotting. In a typical ELISA assay, antigens are attached to a surface. Thus, in the present invention, oxidised CII such as ROS-CII or fragments of CII, for example fragments of CII modified by ROS (ROS-fCII) are attached to a surface. A specific antibody (the primary antibody) is then applied over the surface and binds to the antigen. In this invention, the antibody is in the sample taken from the patient, so the sample is applied over the surface in this step of the method. A second antibody (the secondary antibody) is added, which binds to the primary antibody. The secondary antibody is linked to an enzyme, and then a substance containing the substrate of the enzyme is added. The subsequent reaction produces a detectable signal, typically a colour change in the substrate. The strength of the signal is indicative of the amount of the primary antibody. When the detectable signal is a colour change, a spectrometer is often used to give quantitative values for colour strength.

In one embodiment, an assay in accordance with the first aspect of the invention comprises:
contacting a sample from a subject with oxidized CII;
adding a labeled antibody that binds to anti-oxidised CII antibodies to form a complex; and detecting the formation of a complex between anti-oxidised CII antibodies and labeled antibody;
wherein the detection of said complex is indicative of RA in the subject.

An antibody-antigen binding assay such as an ELISA assay for use in the present invention is carried out using oxidised CII, for example ROS-CII, as a target for the antibodies in the sample taken from the subject. In such an assay, CII can be prepared as the antibody target by chemical modification, for example using a ROS such as HOCl, ONOO⁻, ˙OH or using ribose, to produce oxidised CII. In a suitable ELISA assay, ELISA plates are coated with oxidised CII as bait to bind auto-antibodies from samples, for example SF or serum samples. Samples are then added to the ELISA plates, optionally with buffer added. Optionally, a non-reacting protein can then be added to block any surface of the ELISA plate that remains uncoated with oxidised CII. An enzyme such as horseradish peroxidase can then be added. The enzyme is typically conjugated to an anti-human IgG antibody for binding to the antibodies from the sample. Finally, a substrate, for example a chromogenic substrate such as 3,3',5,5'-tetramethylbenzidine, is added. Activity can then be determined using a suitable method, for example by measuring the optical density (OD). Alternatively, fluorogenic or electrochemiluminescent reporters can be used as appropriate. Suitable ELISA assays for oxidised CII are described in the Examples herein and in Nissim et al. Arthritis & Rheumatism 52: 3829-38, 2005.

Other suitable assays for oxidised CII include: the multiplex assays available from Meso Scale Discovery which are based on the MULTI-ARRAY® technology and allow the assaying of many different antigens (for example CII with different modifications) at the same time; multiplexed bead-based flow cytometry, for example the BD™ Cytometric Bead Array (CBA); and surface plasmon resonance (SPR) based systems, available for example from Biacore.

In some embodiments of the invention, the sample from the subject is compared to a control or a control sample. The control sample is typically a sample taken from a subject who is known not to be suffering from RA, for example a healthy control subject. However, the control sample can also be, for example, from an ACPA positive individual presenting with arthralgia but with no clinical evidence of synovitis or an OA patient, as used in the Examples herein. The control sample can be from a patient with other inflammatory arthritis conditions such as psoriatic arthritis, systemic lupus erythematosus (SLE), ankylosing spondylitis, palindromic arthritis, scleroderma, Behçet's disease, primary Sjögren's syndrome, fibromyalgia, inflammatory arthritis, tendonitis or reactive arthritis. In further embodiments, reference positive and/or negative control samples are used. In other embodiments, native CII (i.e. CII that has not been oxidised) is used as a control. In other embodiments, native human serum albumin (HSA) or bovine serum albumin (BSA) or ROS modified BSA or HSA are used as control antigens.

In the method of the first aspect of the invention and the methods of the other aspects of the invention described herein, the presence or absence of antibodies against oxidised collagen II (CII) can be determined by comparison to a control or a control sample, typically a control sample that is known not to contain antibodies against oxidised CII. If the sample from the subject contains significantly higher levels of antibodies against oxidised CII or fragments thereof than in the control sample, this confirms the presence of antibodies against oxidised CII in the subject. Conversely, if the sample from the subject does not contain significantly higher levels of antibodies against oxidised CII or fragments thereof than in the control sample, this confirms the absence of antibodies against oxidised CII in the subject.

The presence of antibodies against oxidised CII or fragments thereof can be determined, for example, by finding a significant difference between the amount of antibody in the sample versus a control sample. Statistical tests known in the art can be used, for example the Wilcoxon signed rank sum test or the Mann-Whitney test.

The subject is typically a human subject. However, the methods of the invention also find use in the field of veterinary medicine and can therefore be used to diagnose rheumatoid arthritis in animal subjects, typically mammalian subjects, for example companion animals such as cats and dogs, or agricultural animals such as horses, cows and sheep.

The methods of the invention are typically carried out on a sample that has previously been obtained from a subject. Thus, the taking of the sample does not typically form part of the methods of the invention and the methods of the invention are carried out on a sample that has been obtained from a subject. In some embodiments of the invention, however, the method also comprises taking the sample from the subject, for example by taking a blood sample or a synovial fluid sample, a sample of infiltrating immune cells or a cartilage or synovial membrane biopsy. A synovial fluid sample can be collected, for example, during knee arthroscopy or by knee joint aspiration.

The inventors have also surprisingly discovered that there is a correlation between reactivity to oxidised CII and response to disease-modifying anti-rheumatic drugs (DMARDs). In particular, patients who do not react to oxidised CII (i.e. those without auto-antibodies against oxidised CII) respond well to DMARD treatment. Auto-antibodies against oxidised CII can therefore be used as an indication of whether a patient responds to treatment with DMARDs. Accordingly, in a second aspect, the present invention provides a method for identifying whether a subject responds to a disease modifying anti-rheumatic drug (DMARD), comprising:
testing a sample from the subject for the presence or absence of antibodies against oxidised collagen II;
wherein the absence of antibodies against oxidised collagen II in the sample indicates that the subject responds to the DMARD and the presence of antibodies against oxidised collagen II in the sample indicates that the subject does not respond to the DMARD.

Disease-modifying anti-rheumatic drugs (DMARDs) are a category of otherwise unrelated drugs defined by their use in rheumatoid arthritis to slow down disease progression. DMARDs include the gold salts auranofin and sodium aurothiomalate, azathioprine (Imuran), the antimalarials chloroquine and hydroxychloroquine (Plaquenil), ciclosporin (Cyclosporin A), leflunomide (Arava), methotrexate (MTX), minocycline (Minocin), penicillamine, and sulfasalazine (SSZ, Azulfidine). In one embodiment, the DMARD is methotrexate.

The method of the second aspect of the invention is for identifying whether a subject responds to such a DMARD. By "respond to a DMARD" is meant respond to treatment with DMARDs, in other words the use of DMARDs reduce the severity of the symptoms of RA, or eliminate RA altogether, in the patient. Response to treatment with a DMARD can be determined by assessing the disease severity before and after treatment with the DMARD. For example, disease severity can be assessed using the disease activity score (DAS), as described in Fransen et al, Arthritis & Rheumatism (Arthritis Care & Research), vol. 49, no. 5S, pp S214-S224, 2003. The DAS combines single measures into an overall, continuous measure of RA disease activity. A subject who responds to treatment with a DMARD can be classified as one achieving low disease activity, as determined for example using the DAS, after treatment with a DMARD. A subject who does not respond to treatment with a DMARD can be classified as one in which disease activity remains high, as determined for example using the DAS, after treatment with a DMARD.

This method of the second aspect of the invention is typically carried out on a sample from a subject who is known to have RA. Accordingly, in one embodiment of the second aspect of the invention, the subject has RA. For example, the method can be carried out on a subject who has been identified as having RA using a method of the first aspect of the invention. In some embodiments therefore, the method of the second aspect of the invention is carried out in combination with the method of the first aspect of the invention. In another embodiment, the subject is suspected of having RA, for example because the subject exhibits one or more symptoms of RA such as joint pain (arthralgia), musculoskeletal pain, swollen joints (for example joint effusion), synovitis (inflammation of the synovial membrane), stiffness in the joints, fatigue, irritability, depression, anaemia and/or flu-like symptoms.

The method of the second aspect of the invention is also typically carried out on a sample from a subject that has previously been treated or is currently being treated with one or more DMARDs. In this aspect, the present invention relates to a method of monitoring whether a patient is responding to therapy with one or more DMARDs. In an alternative aspect, the subject is not and has not been treated with one or more DMARDs. In this aspect, the present invention relates to a method of predicting whether a patient will respond to therapy with one or more DMARDs.

In the second aspect of the invention, the sample is typically a sample of synovial fluid (SF).

The method of the second aspect of the invention gives an indication of whether a patient responds to a DMARD. Therefore also described herein is a method which comprises a further step of treating a patient with a DMARD. In this embodiment, a determination is made that the subject does not have antibodies against oxidised collagen II and will therefore respond to treatment with the DMARD, and then the subject is treated (or further treated) with the DMARD. Typically in the second aspect of the invention the subject has previously been treated or is currently being treated with one or more DMARDs. Accordingly, this aspect of the invention provides an indication of a successful treatment protocol for a patient with RA. This aspect of the invention can also be utilised when the converse is found, i.e. the test shows the presence of antibodies in the sample, meaning that the patient has auto-antibodies against oxidised CII. This indicates that the patient will not respond to (further) treatment with DMARD and should therefore be treated with an alternative drug.

The non-DMARD RA drug is typically a biologic. By "biologic" is meant a drug or medicinal preparation that is created by a biological process (rather than being chemically synthesized). In one embodiment the biologic is an antibody. Suitable non-DMARD RA drugs for use in accordance with this aspect of the invention include abatacept (Orencia), adalimumab (Humira), anakinra (Kineret), certolizumab pegol (Cimzia), etanercept (Enbrel), golimumab (Simponi), infliximab (Remicade), rituximab (Rituxan), and tocilizumab (Actemra). The first line of biologic treatments are typically anti-TNF (tumour necrosis factor) antibodies, or fragments thereof. Anti-TNF biologies include adalimumab (Humira), certolizumab pegol (Cimzia), etanercept (Enbrel), golimumab (Simponi) and infliximab (Remicade). Biologics which work in a different way, i.e. that do not have TNF as their target, include abatacept (Orencia), anakinra (Kineret), rituximab (Rituxan) and tocilizumab (Actemra).

Typically in this aspect of the invention the subject has previously been treated or is currently being treated with one or more DMARDs.

These aspects of the invention can alternatively be worded as follows.

A DMARD for use in a method of treating rheumatoid arthritis (RA) in a subject in need thereof, wherein the method comprises:
testing a sample from the subject for the presence or absence of antibodies against oxidised collagen II;
identifying the absence of antibodies against oxidised collagen II in the sample; and
administering a DMARD to the subject.

Use of a DMARD in the manufacture of a medicament for the treatment of rheumatoid arthritis (RA) in a subject in need thereof by a method comprising:
testing a sample from the subject for the presence or absence of antibodies against oxidised collagen II;
identifying the absence of antibodies against oxidised collagen II in the sample; and
administering a DMARD to the subject.

A non-DMARD RA drug for use in a method of treating rheumatoid arthritis (RA) in a subject in need thereof, wherein the method comprises:
testing a sample from the subject for the presence or absence of antibodies against oxidised collagen II;
identifying the presence of antibodies against oxidised collagen II in the sample; and
administering a non-DMARD RA drug to the subject.

Use of a non-DMARD RA drug in the manufacture of a medicament for the treatment of rheumatoid arthritis (RA) in a subject in need thereof by a method comprising:
testing a sample from the subject for the presence or absence of antibodies against oxidised collagen II;
identifying the presence of antibodies against oxidised collagen II in the sample; and
administering a non-DMARD RA drug to the subject.

The present inventors have also found that levels of auto-antibodies against oxidised collagen II are high in patients who do not respond to anti-TNF treatment. Auto-antibodies against oxidised collagen II can therefore be used as an indication of whether a patient responds to treatment with anti-TNF biologics.

Accordingly, there is described herein a method for identifying whether a subject responds to an anti-TNF biologic, comprising:
testing a sample from the subject for the presence or absence of antibodies against oxidised collagen II;
wherein the absence of antibodies against oxidised collagen II in the sample indicates that the subject responds to the anti-TNF biologic.

The described method is for identifying whether a subject responds to an anti-TNF biologic. By "respond to an anti-TNF biologic" is meant respond to treatment with an anti-TNF biologic, in other words the use of an anti-TNF biologic reduces the severity of the symptoms of RA, or eliminates RA altogether, in the patient. Response to treatment with an anti-TNF biologic can be determined by assessing the disease severity before and after treatment with the anti-TNF biologic. This can be done as described herein in relation to DMARDs.

This described method is typically carried out on a sample from a subject who is known to have RA. The method is also typically carried out on a sample from a subject that has previously been treated or is currently being treated with one or more anti-TNF biologics. The subject may also have previously been treated with a DMARD. For example, the subject may have been identified as a DMARD non-responder using a method of the second aspect of the invention.

Anti-TNF biologics include adalimumab (Humira), certolizumab pegol (Cimzia), etanercept (Enbrel), golimumab (Simponi) and infliximab (Remicade).

The method gives an indication of whether a patient responds to an anti-TNF biologic. Therefore also described herein is a method which comprises a further step of treating a patient with an anti-TNF biologic. In this embodiment, a determination is made that the subject does not have antibodies against oxidised collagen II and will therefore respond to treatment with an anti-TNF biologic, and then the subject is treated (or further treated) with an anti-TNF biologic. Typically the subject has previously been treated or is currently being treated with one or more anti-TNF biologics. Accordingly, this method provides an indication of a successful treatment protocol for a patient with RA.

This method can also be utilised when the converse is found, i.e. the test shows the presence of antibodies in the sample, meaning that the patient has auto-antibodies against collagen II. This indicates that the patient will not respond to (further) treatment with the anti-TNF biologic and should therefore be treated with an alternative anti-RA biologic which does not act by blocking TNF, i.e. a non-anti-TNF biologic.

Non-anti-TNF biologics, i.e. those that do not have TNF as their target, include abatacept (Orencia), anakinra (Kineret), rituximab (Rituxan) and tocilizumab (Actemra).

Typically in this aspect of the invention the subject has previously been treated or is currently being treated with one or more anti-TNF biologics.

These methods can alternatively be worded as follows.

An anti-TNF biologic for use in a method of treating rheumatoid arthritis (RA) in a subject in need thereof, wherein the method comprises:
testing a sample from the subject for the presence or absence of antibodies against oxidised collagen II;
identifying the absence of antibodies against oxidised collagen II in the sample; and
administering an anti-TNF biologic to the subject.

Use of an anti-TNF biologic in the manufacture of a medicament for the treatment of rheumatoid arthritis (RA) in a subject in need thereof by a method comprising:
testing a sample from the subject for the presence or absence of antibodies against oxidised collagen II;
identifying the absence of antibodies against oxidised collagen II in the sample; and
administering an anti-TNF biologic to the subject.

An anti-RA biologic which does not act by blocking TNF for use in a method of treating rheumatoid arthritis (RA) in a subject in need thereof, wherein the method comprises:
testing a sample from the subject for the presence or absence of antibodies against oxidised collagen II;
identifying the presence of antibodies against oxidised collagen II in the sample; and
administering an anti-RA biologic which does not act by blocking TNF to the subject.

Use of an anti-RA biologic which does not act by blocking TNF in the manufacture of a medicament for the treatment of rheumatoid arthritis (RA) in a subject in need thereof by a method comprising:
testing a sample from the subject for the presence or absence of antibodies against oxidised collagen II;
identifying the presence of antibodies against oxidised collagen II in the sample; and
administering an anti-RA biologic which does not act by blocking TNF to the subject.

The result of these methods is that the RA in the subject is treated using the DMARD or the non-DMARD RA drug such as a biologic, for example an anti-TNF biologic or non-anti-TNF biologic as described in relation to the third aspect of the invention. By "treated" is meant that the severity of the symptoms of RA are reduced, or eliminated altogether in the subject. RA disease severity can be assessed using the disease activity score (DAS), as described herein,

The method of treatment can be of a human or animal subject and this aspect of the invention extends equally to uses in both human and veterinary medicine. The DMARD or the non-DMARD RA drug is preferably administered to a subject in a "therapeutically effective amount", this being sufficient to show benefit to the subject and/or to ameliorate, eliminate or prevent one or more symptoms of RA. As used herein, "treatment" includes any regime that can benefit a human or a non-human animal, preferably a mammal. The treatment may be in respect of an existing condition or may be prophylactic (preventative treatment). The treatment is typically administered to a subject or patient "in need thereof', i.e. a subject suffering from RA.

In this embodiment, the DMARD or the non-DMARD RA drug can be administered to the subject by any appropriate route, for example by oral (including buccal and sublingual), nasal, topical (including transdermal) or parenteral (including subcutaneous, intramuscular, intravenous, intraperitoneal and intradermal) administration, although it will typically be administered to the subject by oral administration. The DMARD or the non-DMARD RA drug can be formulated using methods known in the art of pharmacy, for example by admixing the DMARD or the non-DMARD active ingredient with carrier(s) or excipient(s) under sterile conditions to form a pharmaceutical composition. Accordingly, in one embodiment the subject is administered a pharmaceutical composition comprising a DMARD or a non-DMARD RA drug and one or more carriers and/or excipients.

The DMARD or the non-DMARD RA drug can also be administered in combination with one or more other therapeutically active agents, for example one or more other DMARD or non-DMARD anti-rheumatic drugs. Accordingly, the pharmaceutical composition for use in accordance with this aspect of the invention may also comprise one or more other therapeutically active agents in addition to a DMARD or a non-DMARD RA drug such as a biologic.

Dosages of the DMARD, the non-DMARD RA drug and/or pharmaceutical composition for use in the present invention can vary between wide limits, depending for example on the particular DMARD or the non-DMARD RA drug used, the age and disease stage of the patient, and a physician will ultimately determine appropriate dosages to be used.

The dosage can be repeated as often as appropriate. If side effects develop, the amount and/or frequency of the dosage can be reduced, in accordance with normal clinical practice.

Preferred features for the second aspect of the invention are as for the first aspect *mutatis mutandis.*

The present invention will now be further described by way of reference to the following Examples which are present for the purposes of illustration only. In the Examples, reference is made to a number of Figures in which:

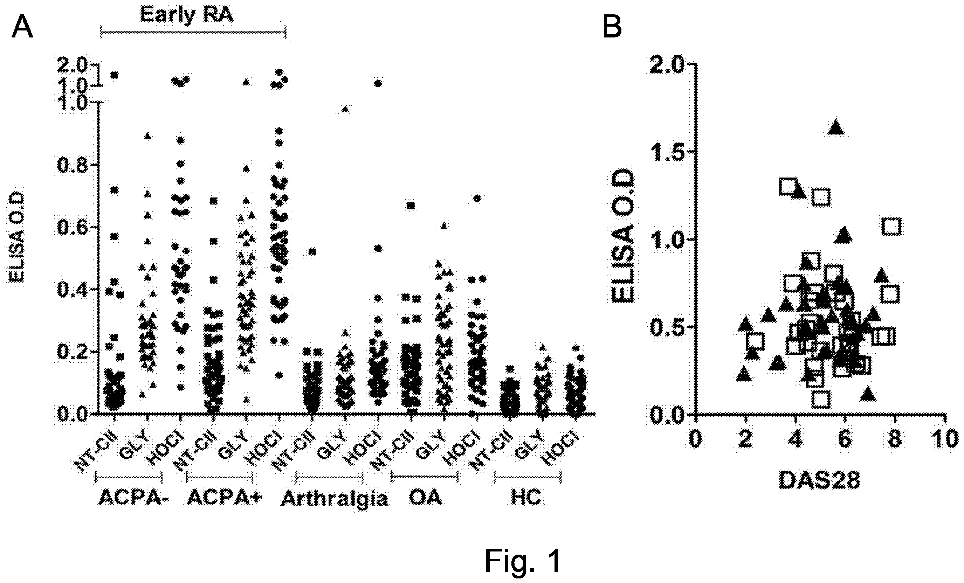

**Figure 1** shows binding to ROS-CII in serum samples from early RA versus control arthralgia, osteoarthritis (OA) and healthy control (HC). **A.** Early RA samples were taken from patients less than 12 months after diagnosis and before the use of any DMARD. Samples were grouped according to the presence of antibodies to citrullinated peptide (ACPA): ACPA positive (ACPA+, n=49) and ACPA negative (ACPA-, n=36). Reactivity in the early RA patients was significantly higher than in arthralgia, OA and HC (p<0.0001). In addition, regardless of ACPA + or ACPA-, reactivity to ROS-CII was significantly higher than to native CII (p<0.001). NT-CII is native CII, GLY is CII modified by ribose and HOCl is CII modified by HOCl. **B**. No correlation between levels of anti-ROS-CII reactivity and levels of DAS28 were observed whether ACPA positive (black triangle) or ACPA negative (white square) (ρ=0.03, ρ=-0.10, for ACPA+ and ACPA-, respectively; p>0.58).

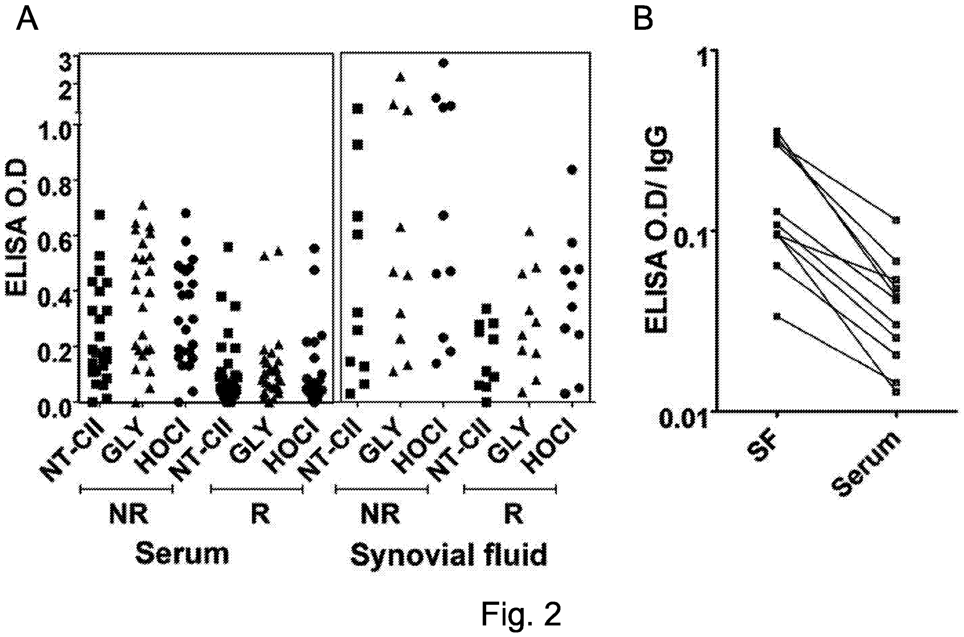

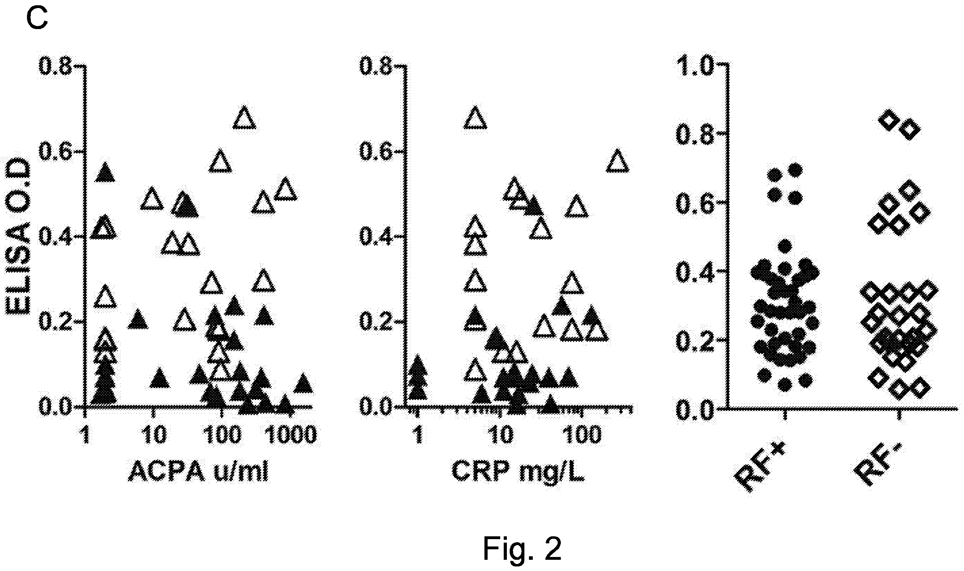

**Figure 2** shows anti-ROS-CII reactivity in samples from established RA patients. **A.** Serum and synovial fluid (SF) samples from patients with established RA were grouped into those patients who responded to DMARD (DMARD-R) and patients who did not respond to DMARD (DMARD-NR). Higher reactivity was observed in DMARD-NR (p<0.01) in both serum and SF samples. Higher ROS-CII auto-response in SF was also observed compared to serum (p<0.05). NT-CII is native CII, GLY is CII modified by ribose and HOCl is CII modified by HOCl. **B**. Anti-ROS-CII reactivity was tested in matched SF and serum samples. Levels of anti-ROS-CII reactivity were normalised to total levels of IgG in matched serum and SF. Higher reactivity in SF was seen (p=0.001). **C.** No correlation between levels of anti-ROS-CII reactivity and levels of ACPA (ρ=0.32, ρ=-0.22, for DMARD-NR or DMARD-R, respectively; p>0.134) and CRP (ρ=0.08, ρ=0.12, for DMARD-NR or DMARD-R, respectively; p>0.74) were observed whether DMARD-R (black triangle) or DMARD-NR (white triangle). In addition, reactivity in RF positive patients (black circle, RF+) was similar to reactivity in RF negative samples (white diamond, RF-, p>0.05).

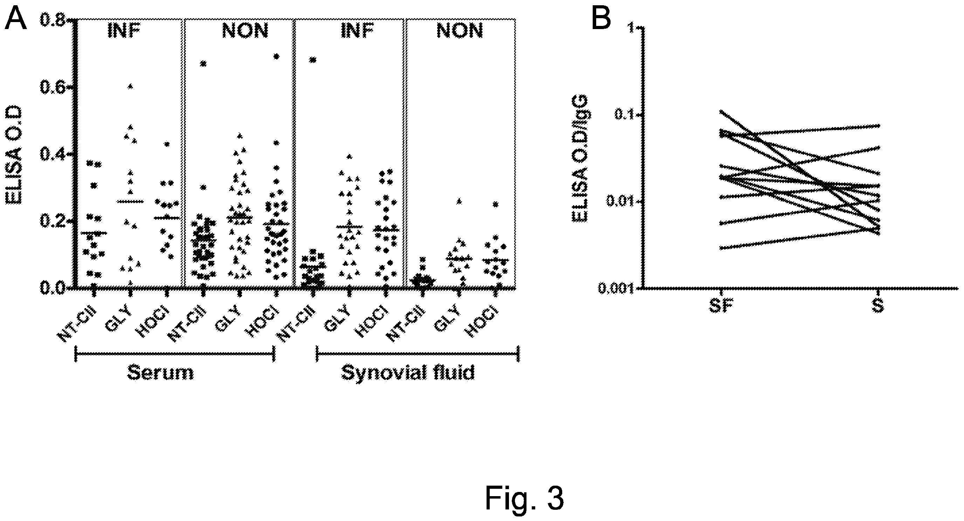

**Figure 3** shows anti-ROS-CII reactivity in serum and synovial fluid (SF) from patients with osteoarthritis (OA). **A**. The binding to ROS-CII in OA was distinct from RA with tendency for higher reactivity in the serum. Samples from inflammatory (INF) OA had higher reactivity to ROS-CII compared to non-inflammatory (NON) OA (p<0.005 for SF and p=0.05 for serum). **B.** Matched OA SF and serum samples displayed no significant difference in reactivity (p=0.272), with samples displaying either higher or lower reactivity in SF versus serum. Levels of anti-ROS-CII reactivity in matched serum and SF were normalised to their respective total levels of IgG.

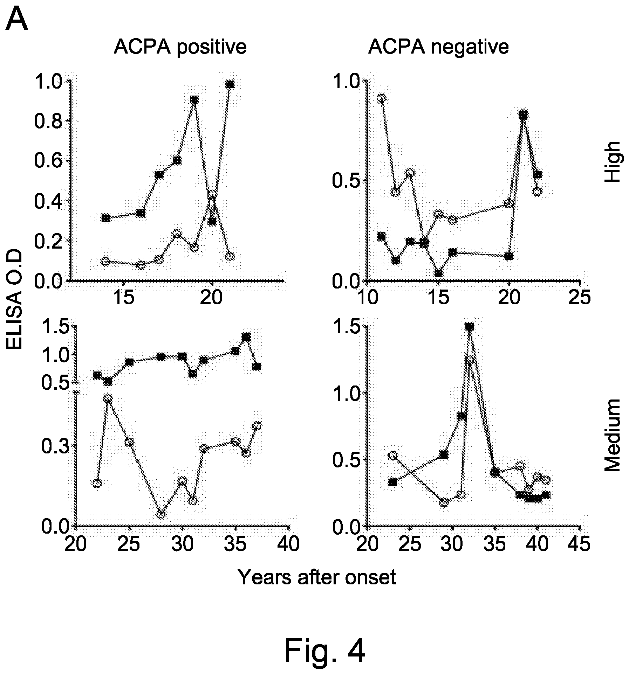

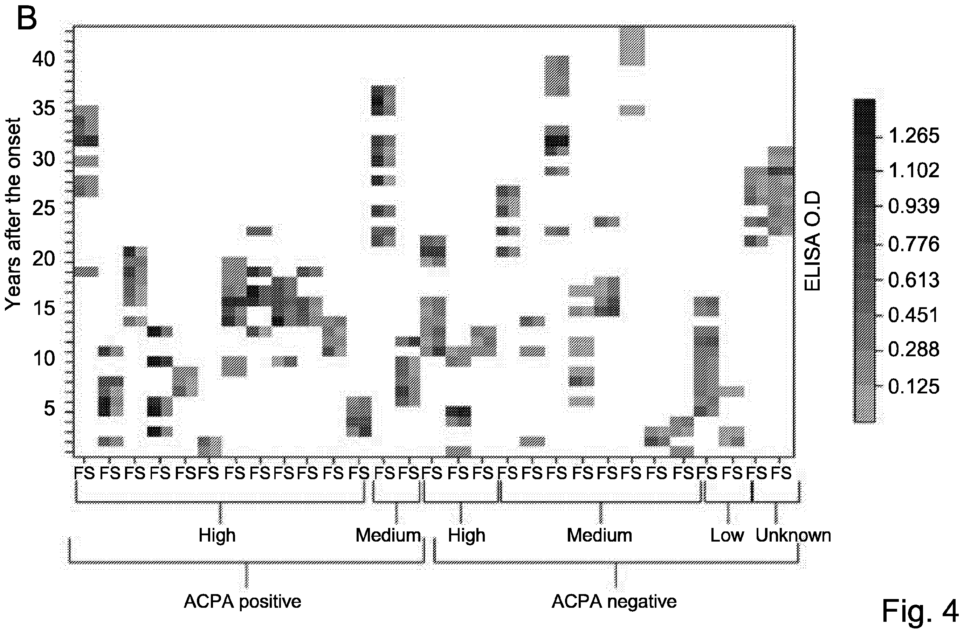

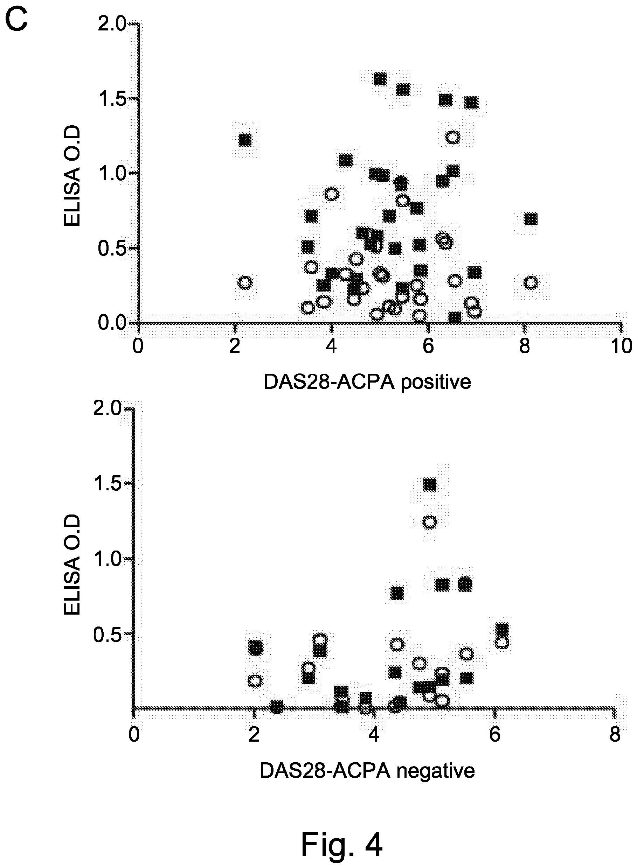

**Figure 4** shows longitudinal follow up of anti-ROS-CII auto-reactivity in chronic longstanding RA. **A.** Anti-ROS-CII reactivity in four different patients is shown as examples to demonstrate the longitudinal changes. Reactivity in SF (black square) is higher than in serum (white circle) in the ACPA positive samples but similar or lower compared to serum in the ACPA negative samples. High and medium are patients with high or medium disease activity, respectively. **B.** ELISA O.D for all tested samples is displayed as a gradient from black to light grey colour representing the highest and lowest OD, respectively. Matched SF and serum samples collected from each patient are shown as pairs (F represents the SF and S the serum). In the SF a trend for higher OD was observed in the ACPA positive group but not in ACPA negative group. C. No correlation between DAS28 and anti-ROS-CII reactivity was observed in both serum (white circle) and SF (black square) in both ACPA positive and ACPA negative patients (ρ=0.3006; p=2173 and ρ=0.31718; p=0.1736 for SF and serum from ACPA negative and ρ=0.04581; p=0.8134 and ρ=0.0696; p=0.5346 for serum and SF from ACPA positive patients). Patients were grouped into high (DAS28>5.1), medium (3.2<DAS28<5.1), low (2.6<DAS28<3.2) disease activity and with 2 unknown.

### Example 1

### Patients and Methods

### Patients and clinical samples

Serum samples were collected from the following centres: Karolinska Institute, Sweden; Leeds Division of Rheumatology and Musculoskeletal diseases, UK; Barts Hospital in London, UK; Kennedy Institute of Rheumatology, UK and University of Pavia School of Medicine, Italy. Patients were defined by ACR 1987 criteria and the diagnosis was made by a specialist rheumatologist. Ethical approval was obtained from all clinical centres involved, and informed consent was obtained from all individuals prior to collecting blood or synovial fluid (SF) samples. SF samples were collected during knee arthroscopy or directly by knee joint aspiration.

### Tested samples were categorised as follows:

1) Disease Modifying Anti Rheumatic Drug **(DMARD)-naïve early RA** patients, with <12 months symptom duration. Bloods were taken at the first visit to a specialist clinic and before the use of any DMARD (n=85 serum samples). Patients were either ACPA positive (n=49) or ACPA negative (n=36).
2) Samples from established RA patients with a disease duration of more than one year that were grouped into: 2a) **DMARD responders (DMARD-R):** achieving low disease activity (LDAS<3.2) after treatment with DMARD (n=26 serum and n=10 SF); and 2b) **DMARD non-responders** (DMARD-NR) with DAS remaining ≥3.2 despite treatment (n=24 serum and n=10 SF).
3) Longitudinal follow up of established longstanding RA: matched SF and serum from 30 established RA patients who were followed longitudinally for up to forty three years (2-11 samples per patient). 15 patients were ACPA positive and 15 were ACPA negative. Patients were grouped into individuals with high disease activity with DAS28≥5.1; medium with 3.2≤DAS28≤5.1 and low disease activity with DAS28≤3.2. Most patients were already treated with biologics at the time of sampling.

Blood samples and DAS28 measurement were taken in the clinic at the time of visit for group 2 and 3.

### Control groups included:

4) ACPA positive individuals presenting with arthralgia but with no clinical evidence of synovitis (n=58 serum samples). Individuals were recruited from patients with new musculoskeletal pain complaints (usually involving 1 or 2 joint) or arthralgia. When patients were ACPA positive by the CCP-2 test, they were seen by an experienced rheumatologist, who established the absence of clinical evidence of synovitis. Normal levels of CRP<10 mg/L (according to local range) was also confirmed. 11 out of 58 individuals had signs of OA but were followed for 36 months with no sign of synovitis.
5) OA serum samples (n=49) and OA SF (n=52) from patients with ACR criteria of OA knee (Altman et al., Arthritis & Rheumatism 29(8): 1039-1049, 1986). When detailed clinical information was available, OA patients were classified as inflammatory or not according to the symptoms and the presence or absence of clinical synovitis and/or joint effusion. Patients with severe knee pain during any level of physical activity and which disturbed sleep on a daily basis, or who had persistent joint effusion despite intra-articular steroid and oral antiinflammatories were classed as inflammatory. Patients with only intermittent mild knee pain and/or swelling which responded to quadriceps strengthening exercises and/or paracetamol were classed as non-inflammatory OA.
6) Sex and age matched serum samples from healthy individuals (HC, n=51) were collected from a range of volunteers with no inflammatory joint disease reported. OA may be presented in a few older individuals but with no required medication to alleviate their symptoms. CCP-2 test was negative in all HC individual (rheumatoid factor (RF) data was not available).

### Enzyme-linked immunoabsorbent assay (ELISA)

CII was chemically modified as previously described to generate CII post-translationally modified by HOCl, ONOO⁻, ˙OH or ribose (Nissim et al. Arthritis & Rheumatism 52: 3829-38, 2005). Bovine serum albumin (BSA, Sigma) and human serum albumin (HSA, Sigma) were similarly modified and were used as control antigens. The results section shows the data for glycated CII (Gly-CII) and CII modified by HOCl (HOCL-CII) as an example for ROS-CII in comparison to native CII (NT-CII).

An ELISA was performed using the ROS-CII or native CII as targets as described previously (Nissim et al. Arthritis & Rheumatism 52: 3829-38, 2005). Briefly, ELISA plates were coated with 10µg/ml of ROS-CII or native CII as bait to bind auto-antibodies from SF or serum samples. The ELISA optical density measurement (OD) obtained for BSA, ROS-modified BSA (ROS-BSA), HSA, and ROS-modified HSA (ROS-HSA) were used as a background control to normalise the respective ELISA-OD for native and ROS-CII. In addition, to control the assay fluctuation we performed the ELISA using the same batch of modified-CII for any groups that were going to be compared. Each assay included a known reference positive or negative control sample. Longitudinal samples from the same individual (serum and SF) were tested on the same day using the same batch of ROS-CII.

In the absence of absolute standards (as for the CCP2 kits), titres could not be measured by the ELISA. Arbitrary OD values were therefore used. Patients positive for anti-ROS-CII auto-antibodies (later abbreviated as "binders") were therefore defined using the 95^{th} percentile of the healthy controls ELISA OD plus 2 standard deviation (SD) as a cut-off set to OD=0.28.

When paired serum and SF were tested, arbitrary ELISA OD units were normalised to their respective IgG levels. IgG levels were measured using Human IgG ELISA Quantitation set (Cambridge Bioscience, Cambridge, UK) following the manufacturer's instructions.

An ACPA ELISA was performed using the anti-CCP2 test kit (UK samples) and according to the manufacturer's instructions using Axis-Shield Diagnostics Limited, (Dundee, UK) or Eurodiagnostica, (Malmo, Sweden, for samples obtained from Sweden). The decision as to a positive or negative result to the CCP-2 test followed the local clinical practice (UK and Sweden) according to standard of good clinical practice at each centre.

### Statistical analysis

Variables were not normally distributed, therefore non-parametric tests were used. The Wilcoxon signed rank sum test was used to compare the reactivity between native and ROS-CII, while Mann-Whitney tests were used to compare between the various groups. Correlation was measured using Spearman test, a nonparametric correlation test. To determine diagnostic discrimination between early RA, arthralgia, OA and HC, we used the cut-off point of 0.28 OD units to construct a contingency table of positive autoantibodies against clinical diagnosis (early RA versus healthy control; early RA versus arthralgia; early RA versus OA) and tested it by Fisher's Exact Test. A nonparametric Wilcoxon-type test for trend was used to test the longitudinal follow up of anti-ROS-CII reactivity across for each individual. Statistical analysis was performed using the GraphPad Prism software package (GraphPad Software, San Diego, CA) and Stata 12 (StataCorp. 2011. Stata Statistical Software: Release 12. College Station, TX: StataCorp LP).

### RESULTS

### Binding to ROS-CII in samples from early RA versus HC, arthralgia and OA

Reactivity in DMARD naive early RA to ROS-CII was significantly higher than in ACPA positive arthralgia, OA and HC (Fig.1, p<0.0001) and irrespective of ACPA status with 90.5% binders to HOCl-CII and 61% to Glycated CII (Table 1). This suggests that a routine test for RA using ROS-CII as an ACPA-dependent biomarker would have high detection power.

In contrast, binding to native CII was significantly lower than binding to ROS-CII (p<0.0001) with only 18.8% binders. There was no significant difference (p>0.05) in binding to ROS-CII between the DMARD naive early RA ACPA positive (n=49) and ACPA negative (n=36) with 93% and 86% binders to HOCl-CII, respectively (Fig. 1, Table 1). Reactivity to glycated CII was however slightly higher in ACPA positive than in ACPA negative with 71% versus 47% binders (p=0.024). In ACPA positive arthralgia, we observed significantly higher binding to HOCl-CII than in healthy controls (p<0.001) with 6.8% binders, while only 1.7% bound to glycated or native CII (Fig 1). Although patients with OA had significantly lower reactivity in comparison to early RA (p<0.001), 30.6% bound to glycated CII (Fig.1), but only 14.2% bound to HOCl-CII (Table 1). There were no significant gender or age differences between the HC versus OA, arthralgia and early RA DMARD naive patients (Table 1).

**Table 1. Distribution of binders to ROS-CII**

| DMARD-R: established RA patients that respond to DMARD; DMARD-NR: established RA patients that did not respond to DMARD; SF: synovial fluid; Inflammatory OA is severe OA and non-inflammatory OA is mild OA. | | | | | | | |
|---|---|---|---|---|---|---|---|
| | **Group** | **n** | **Age** | **Gender (% F**) | **Binders (%)** | | |
| | | | | | **NT-CII** | **GLY-CII** | **HOCl-CII** |
| **RA serum** | **Early RA** | 85 | 51 (22-70) | 72 | 18.8 | 61.0 | 90.5 |
| | **Early RA ACPA-** | 36 | 53 (29-70) | 66 | 19.4 | 47.2 | 86.1 |
| | **Early RA ACPA+** | 49 | 48 (22-69) | 78 | 18.3 | 71.4 | 93.8 |
| | **DMARD-R** | 26 | 55 (23-84) | 76 | 7.6 | 7.6 | 7.6 |
| | **DMARD-NR** | 24 | 56 (31-81) | 75 | 37.5 | 58.3 | 54.1 |
| **RA SF** | **DMARD-R** | 10 | 46 (21-75) | 80 | 20.0 | 40.0 | 50.0 |
| | **DMARD-NR** | 10 | 48 (48-71) | 100 | 50.0 | 70.0 | 70.0 |

| **Controls** | | | | | | | |
|---|---|---|---|---|---|---|---|
| **Arthralgia serum** | **ACPA positive** | 58 | 53 (27-76) | 83 | 1.7 | 1.7 | 6.8 |
| **OA serum** | **OA** | 49 | 61.5 (27-81) | 72 | 6.1 | 30.6 | 14.2 |
| | **no synovitis** | 35 | 61 (27-81) | 67 | 0.0 | 25.7 | 11.4 |
| | **with synovitis** | 14 | 65 (29-75) | 78 | 21.4 | 42.8 | 14.2 |
| **OA SF** | **no synovitis** | 36 | 60 (39-89) | 50 | 0.0 | 0.0 | 0.0 |
| | **with synovitis** | 16 | 58 (43-78) | 50 | 4.1 | 20.8 | 12.5 |
| **Healthy serum** | **Healthy** | 51 | 48 (26-70) | 66 | 0.0 | 0.0 | 0.0 |

The sensitivity and specificity of the binding of autoantibodies to ROS-CII (here defined as both glycated CII and HOCl-CII) in early RA compared with HC was 90% and 100%, respectively (OR 936 [95% CI 53-16639], p<0.0001); for early RA compared with arthralgia sensitivity and specificity was 90% and 93%, respectively (OR 129.9 [95% CI 37.2-453.5], p<0.0001). In respect to OA, specificity and sensitivity for anti-CII-HOCI reactivity were 85% and 90% (OR 57.75 [95% CI 19.7-170.4], p<0.0001). However, the reactivity to glycated CII was less specific in early RA compared to OA with sensitivity of and specificity of 61% and 67%, respectively. (OR 3.25 [95% CI 1.5-6.8], p=0.002).

In order to examine whether the presence of auto-reactivity to modified collagen was a novel diagnostic biomarker, we investigated whether autoantibodies were associated with disease activity or levels of inflammation reflected by DAS28 values. There was no relationship between anti-ROS-CII ODs and DAS28 whether ACPA was negative or positive (ρ=0.03, ρ =-0.10, for ACPA+ or ACPA-, respectively; p>0.58, Fig.1B).

### Anti-ROS reactivity in patients with established RA

Despite the similarity in age and sex between the DMARD-R and DMARD-NR groups (Table 1), there was a striking difference in the observed auto-immune-reactivity towards ROS-CII in the tested serum samples (Fig. 2). The strongest reactivity was seen in DMARD-NR with 54% binders to HOCl-CII versus 7.6% in DMARD-R (p<0.01, Table 1). Similarly, SF samples categorised according to patients' response to DMARD displayed the same pattern of reactivity, but it was quite apparent that the levels of anti-ROS-CII auto-response in DMARD-NR and DMARD-R SF was significantly higher than in serum (p<0.05 and p<0.007, respectively), with 70% and 50% binders to HOCl-CII in DMARD-NR and DMARD-R, respectively (Fig. 2A). To confirm that the increased binding in SF was not an artefact related to the difference in levels of immunoglobulin in SF versus blood, we tested a set of paired SF and serum samples where binding to HOCl-CII was normalised according to their corresponding levels of IgG. Increased anti-ROS-CII reactivity in the SF compared to serum was further confirmed in the paired SF and serum samples (Fig. 2B, p=0.001).

Patients were both ACPA positive and negative. No association between anti-ROS-CII reactivity and ACPA status for either DMARD-NR or DMARD-R was observed (ρ=0.32, ρ =-0.22, for DMARD-NR or DMARD-R, respectively; p>0.134) or CRP (ρ=0.08, ρ=0.12, for DMARD-NR or DMARD-R, respectively; p>0.74) (Fig 2C). In addition, reactivity in RF positive patients was similar to the reactivity in the RF negative patients (p>0.05, Fig 2C).

### Anti-ROS-CII binding in inflammatory OA versus non-inflammatory OA

The presence of anti-ROS-CII antibodies was examined in more details in OA with respect to the presence of clinical evidence of synovitis, severe pain or persistent effusion (Table 1, Fig. 3A). Reactivity to HOCl-CII in serum samples from severe inflammatory OA and mild non-inflammatory OA was low with 14.2% and 11.4% binders, respectively. Reactivity to glycated CII was however higher with 42.8% and 25.7% binders to glycated CII in severe inflammatory OA and mild non-inflammatory OA, respectively (Fig 3A, Table 1). In addition, the pattern of binding to ROS-CII in OA was different from RA with a tendency for higher reactivity in the serum than in the SF, the opposite of the situation in RA. In contrast to RA, matched OA SF and serum samples showed no tendency towards higher reactivity in SF and samples displayed either higher or lower reactivity (p=0.272, Fig 3B).

### Longitudinal study of ROS-reactivity in patients with chronic longstanding disease

To further study the correlation between anti-ROS-CII reactivity and disease evolution, we analysed matched SF and serum samples from 30 patients with chronic RA collected longitudinally. As seen in Fig. 4A-B the anti-HOCl-CII reactivity varies considerably. Similar variability was shown also for glycated CII (data not shown). A trend of higher reactivity was observed in the SF of the ACPA positive group compared to the SF of the ACPA negative group. Anti-ROS-CII reactivity in the serum of both groups was, however, similar (Fig. 4B). Nevertheless, no correlation between levels of ROS-CII auto-reactivity and disease activity (DAS28) at the time of sampling was found, (ρ=0.3006; p=2173 and ρ=0.31718; p=0.1736 for SF and serum from ACPA negative patients and ρ=-0.04581; p=0.8134 and ρ=0.0696; p=0.5346 for serum and SF from ACPA positive patients, DAS28 was not available for all tested samples, Fig.4C).

A nonparametric test for trend stratified by individual showed no evidence of a trend in anti-ROS reactivity over time (p=0.634). Similarly, when a multilevel model was fitted to the data, time since diagnosis was not a significant predictor of anti-ROS reactivity, both in the unadjusted model (p=0.834) and when adjusted for ACPA status and DAS category (p=0.631). Only having a high DAS score compared to a low DAS score was a significant predictor of anti-ROS reactivity (p=0.025).

In conclusion, these results show that anti-ROS-CII auto-antibodies provide a novel, serological biomarker that can: a) facilitate RA diagnosis, particularly in the ACPA negative patients; b) lead to better RA subgrouping; c) facilitate prediction of disease outcome and response to DMARD and anti-TNF treatment in RA patients; and d) facilitate OA diagnosis.

## Claims

1. A method of diagnosing rheumatoid arthritis (RA), comprising:
testing a sample from a subject for the presence or absence of antibodies against oxidised collagen II;
wherein the presence of antibodies against oxidised collagen II in the sample is indicative of RA in the subject, and wherein the subject does not have antibodies to cyclic citrullinated peptides (ACPA).

2. A method for identifying whether a subject responds to a disease modifying anti-rheumatic drug (DMARD), comprising:
testing a sample from the subject for the presence or absence of antibodies against oxidised collagen II;
wherein the absence of antibodies against oxidised collagen II in the sample indicates that the subject responds to the DMARD and the presence of antibodies against oxidised collagen II in the sample indicates that the subject does not respond to the DMARD.

3. A method according to claim 2,
(a) wherein the DMARD is selected from the group
consisting of auranofin, sodium aurothiomalate, azathioprine, chloroquine, hydroxychloroquine, ciclosporin (Cyclosporin A), leflunomide, methotrexate (MTX), minocycline, penicillamine, and sulfasalazine (SSZ), and/or
(b) wherein the subject has RA, and/or
(c) wherein the subject has previously been treated or is currently being treated with one or more DMARDs.

4. A method according to any one of the preceding claims, wherein the sample is a sample of serum or plasma or whole blood or synovial fluid.

5. A method according to any one of the preceding claims, wherein the oxidised CII has been oxidised by non-enzymatic glycation or by reactive oxygen species (ROS) or by reactions involving aldehydes.

6. A method according to claim 5,
(a) wherein the ROS is selected from the group consisting of superoxide radical (O₂˙⁻), hydrogen peroxide (H₂O₂), lipid hydroperoxides (LOOH), lipid hydroperoxides in combination with transition metal ions, hydrogen peroxide in combination with transition metal ions, hydroxyl radical (˙OH), hypochlorous acid (HOCl), hypobromous acid (HOBr), nitric oxide (NO˙), nitrogen dioxide (NO₂˙), and peroxynitrite (ONOO⁻), or
(b) wherein glycation is induced by glucose, ribose or by other sugars, or
(c) wherein the aldehydes are selected from the group consisting of malonaldehyde and 4-hydroxynonenal.

7. A method according to any one of the preceding claims, wherein the presence or absence of antibodies against oxidised collagen II is determined using an ELISA assay.

8. A DMARD for use in a method of treating rheumatoid arthritis (RA) in a subject in need thereof, wherein the method comprises:
testing a sample from the subject for the presence or absence of antibodies against oxidised collagen II;
identifying the absence of antibodies against oxidised collagen II in the sample; and
administering a DMARD to the subject.

## Patentansprüche

1. Verfahren zur Diagnose von rheumatoider Arthritis (RA), das Folgendes umfasst:
Testen einer Probe von einem Individuum auf An- oder Abwesenheit von Antikörpern gegen oxidiertes Kollagen II;
wobei die Anwesenheit von Antikörpern gegen oxidiertes Kollagen II in der Probe auf RA bei dem Individuum hinweist, und wobei das Individuum keine Antikörper gegen cyclische citrullinierte Peptide (ACPA) aufweist.

2. Verfahren zur Identifizierung, ob ein Individuum auf ein krankheitsmodifizierendes Antirheumatikum (Disease Modifying Anti-Rheumatic Drug, DMARD) anspricht, das Folgendes umfasst:
Testen einer Probe von einem Individuum auf An- oder Abwesenheit von Antikörpern gegen oxidiertes Kollagen II;
wobei die Abwesenheit von Antikörpern gegen oxidiertes Kollagen II darauf hinweist, dass das Individuum auf das DMARD anspricht, und die Anwesenheit von Antikörpern gegen oxidiertes Kollagen II darauf hinweist, dass das Individuum nicht auf das DMARD anspricht.

3. Verfahren nach Anspruch 2,
(a) wobei das DMARD aus der Gruppe bestehend aus Auranofin, Natriumaurothiomalat, Azathioprin, Chloroquin, Hydroxychloroquin, Ciclosporin (Cyclosporin A), Leflunomid, Methotrexat (MTX), Minocyclin, Penicillamin und Sulfasalazin (SSZ) ausgewählt ist und/oder
(b) wobei das Individuum RA hat und/oder
(c) wobei das Individuum zuvor mit einem oder mehreren DMARDs behandelt wurde oder gegenwärtig mit einem oder mehreren DMARDs behandelt wird.

4. Verfahren nach einem der vorhergehenden Ansprüche, wobei es sich bei der Probe um eine Probe von Serum oder Plasma oder Vollblut oder Synovialflüssigkeit handelt.

5. Verfahren nach einem der vorhergehenden Ansprüche, wobei das oxidierte CII durch nichtenzymatische Glykierung oder durch reaktive Sauerstoffspezies (Reactive Oxygen Species, ROS) oder durch Reaktionen unter Beteiligung von Aldehyden oxidiert wurde.

6. Verfahren nach Anspruch 5,
(a) wobei die ROS aus der Gruppe bestehend aus dem Superoxid-Radikal (O2˙⁻), Wasserstoffperoxid (H2O2), Lipidhydroperoxiden (LOOH), Lipidhydroperoxiden in Kombination mit Übergangsmetallionen, Wasserstoffperoxid in Kombination mit Übergangsmetallionen, dem Hydroxylradikal (˙OH), hypochloriger Säure (HOCl), hypobromiger Säure (HOBr), Stickstoffmonoxid (NO˙), Stickstoffdioxid (NO₂˙) und Peroxynitrit (ONOO⁻) ausgewählt ist oder
(b) wobei die Glykierung durch Glucose, Ribose oder durch andere Zucker induziert wird oder
(c) wobei die Aldehyde aus der Gruppe bestehend aus Malonaldehyd und 4-Hydroxynonenal ausgewählt sind.

7. Verfahren nach einem der vorhergehenden Ansprüche, wobei die An- oder Abwesenheit von Antikörpern gegenüber oxidiertem Kollagen II mit Hilfe eines ELISA-Assays bestimmt wird.

8. DMARD zur Verwendung bei einem Verfahren zur Behandlung von rheumatoider Arthritis (RA) bei einem Individuum, bei dem diesbezüglich Bedarf besteht, wobei das Verfahren Folgendes umfasst:
Testen einer Probe von einem Individuum auf An- oder Abwesenheit von Antikörpern gegen oxidiertes Kollagen II;
Identifizieren der Abwesenheit von Antikörpern gegen oxidiertes Kollagen II in der Probe; und Verabreichen eines DMARD an das Individuum.

## Revendications

1. Méthode de diagnostic de la polyarthrite rhumatoïde (PR), comprenant :
le dosage d'un échantillon issu d'un sujet afin de détecter la présence ou l'absence d'anticorps contre le collagène II oxydé ;
où la présence d'anticorps contre le collagène II oxydé dans l'échantillon constitue une indication de PR chez le sujet, et où le sujet ne possède pas d'anticorps contre les peptides cycliques citrullinés (ACPA).

2. Méthode d'identification si un sujet répond ou non vis-à-vis d'un médicament antirhumatismal modificateur de maladie (DMARD), comprenant :
le dosage d'un échantillon issu d'un sujet afin de détecter la présence ou l'absence d'anticorps contre le collagène II oxydé ;
où l'absence d'anticorps contre le collagène II oxydé dans l'échantillon indique que le sujet répond vis-à-vis du DMARD et la présence d'anticorps contre le collagène II oxydé dans l'échantillon indique que le sujet ne répond pas vis-à-vis du DMARD.

3. Méthode selon la revendication 2,
(a) où le DMARD est choisi dans le groupe constitué par l'auranofine, l'aurothiomalate de sodium, l'azathioprine, la chloroquine, l'hydroxychloroquine, la cyclosporine (cyclosporine A), le léflunomide, le méthotréxate (MTX), la minocycline, la pénicillamine, et la sulfasalazine (SSZ), et/ou
(b) où le sujet souffre de PR, et/ou
(c) où le sujet a déjà été traité ou est en cours de traitement par un ou plusieurs DMARD.

4. Méthode selon l'une quelconque des revendications précédentes, dans laquelle l'échantillon est un échantillon de sérum ou de plasma ou de sang entier ou de liquide synovial.

5. Méthode selon l'une quelconque des revendications précédentes, dans laquelle le CII oxydé a été oxydé par une glycation non enzymatique ou par un dérivé réactif de l'oxygène (ROS) ou par des réactions mettant en jeu des aldéhydes.

6. Méthode selon la revendication 5,
(a) où le ROS est choisi dans le groupe constitué par un radical de superoxyde (O₂˙⁻), le peroxyde d'hydrogène (H₂O₂), les hydroperoxydes lipidiques (LOOH), les hydroperoxydes lipidiques en combinaison avec des ions de métaux de transition, le peroxyde d'hydrogène en combinaison avec des ions de métaux de transition, un radical hydroxyle (˙OH), l'acide hypochloreux (HOCl), l'acide hypobromeux (HOBr), l'oxyde nitrique (NO˙), le dioxyde d'azote (NO₂˙), et le peroxynitrite (ONOO⁻), ou
(b) où la glycation est induite par le glucose, le ribose ou par d'autres sucres, ou
(c) où les aldéhydes sont choisis dans le groupe constitué par le malonaldéhyde et le 4-hydroxynonénal.

7. Méthode selon l'une quelconque des revendications précédentes, dans laquelle la présence ou l'absence d'anticorps contre le collagène II oxydé est déterminée à l'aide d'un dosage ELISA.

8. DMARD destiné à une utilisation dans une méthode de traitement de la polyarthrite rhumatoïde (PR) chez un sujet en ayant besoin, où la méthode comprend :
le dosage d'un échantillon issu du sujet afin de détecter la présence ou l'absence d'anticorps contre le collagène II oxydé ;
l'identification de l'absence d'anticorps contre le collagène II oxydé dans l'échantillon ; et
l'administration d'un DMARD au sujet.
